# EUROPEAN PATENT SPECIFICATION

(11) **EP 2 100 628 B1**
(45) Date of publication and mention of the grant of the patent: **15.04.2015**
(21) Application number: 07849957.1
(22) Date of filing: 30.11.2007
(51) Int. Cl.: A61L 31/04, A61L 31/14, A61L 24/00, A61L 24/08

(54) **SELF-DEGRADABLE ADHESIVE FOR MEDICAL USE OF TWO- COMPONENT REACTANT SYSTEM COMPRISING POWDER-POWDER**
SELBSTABBAUENDER KLEBSTOFF ZUR MEDIZINISCHEN VERWENDUNG EINES ZWEIKOMPONENTEN-REAKTIONSSYSTEMS MIT PULVER-PULVER
ADHÉSIF AUTODÉGRADABLE À USAGE MÉDICAL D'UN SYSTÈME RÉACTIF À DEUX COMPOSANTS COMPRENANT LE MÉLANGE POUDRE-POUDRE

(30) Priority: 30.11.2006 JP 2006323720; 30.07.2007 US 881941
(43) Date of publication of application: 16.09.2009
(73) Proprietor: BMG Incorporated, Kyoto 601-8023 (JP)
(72) Inventor: NAKAJIMA, Naoki, Muko-shi Kyoto 617-0002 (JP); SUGAI, Hajime, Hirakata-shi Osaka 573-0036 (JP); KONDA, Masakazu, Uji-shi Kyoto 611-0041 (JP); HYON, Suong-Hyu, Uji-shi Kyoto 611-0021 (JP)
(74) Representative: Strehl Schübel-Hopf & Partner
(86) International application number: PCT/JP2007/073251
(87) International publication number: WO 2008/066182

(56) References cited:
- WO-A1-00/38752
- WO-A1-2006/080523
- WO-A1-2006/080523
- JP-A- 09 002 971
- JP-A- 11 290 445
- JP-A- 60 204 725
- JP-A- 2001 514 050
- JP-A- 2002 533 164
- JP-A- 2003 126 235
- US-A1- 2005 002 893

## Description

### Field of the Invention

This invention relates to a medical-use adhesive that is used for bonding of living tissues, filling or preventing of adhesion between living tissues, or for stopping of bleeding, after surgical operation or the like, as well as a medical-use resin. Especially, the invention relates to an adhesive, which is comprised of (1) powder containing first reactive component, or powder-form reactive agent, and (2) powder (powder-form reactive agent) containing second reactive component, and which cures to form a gel after mixing of first and second components and then decomposes to be fluidized and excreted after elapse of a certain period.

### Background of the Invention

As an adhesive for medical use, especially for surgical operation; (1) cyanoacrylate adhesives and (2) fibrin glue have been predominantly used. However, cured resin of the cyanoacrylate adhesives has poor flexibility and is rigid and may thereby deter healing of wounds. Moreover, the cured resin has further problems such as being difficult to be decomposed as to be remained as a foreign body after being enveloped. Meanwhile, the fibrin glue has only poor level of adhesive strength so that produced fibrin masses may be occasionally peeled off from the tissue. Moreover, due to being a blood product, there remains a fear of virus transmission.

On the other hand, following adhesives have been recently investigated. (3) Dextranaldehyde (oxidized dextran being a polyaldehyde)/high-molecular-weight chitosan WO 2003/035122 of AESCULAP AG & CO KG (DE); a counterpart of US2005/0002893 A-1 and EP1438079 B1.; (4) Micelle-forming polymer with aldehyde end groups/high-molecular-weight polyallylamine JP-2005-021454A; NISHIDA, Hiroshi; YOKOYAMA, Masayuki, "Tissue adhesive formed of polymer micelle as effective component".; (5) Starch aldehyde (oxidized starch being a polyaldehyde)/collagen WO98/15299; "ADHESIVE COMPOSITION WITH MACROMOLECULAR POLYALDEHYDE BASE AND METHOD FOR CROSS-LINKING COLLAGEN"; a counterpart of Japan's issued patent 323871; (6) Gelatin/succinimidized poly-L-glutamate; (7) Gelatin/dicarboxylic acid; and (8) Urethane polymer. Nevertheless, each of these adhesives has some problems. Please see WO/06/080523 ("Medical-use two-component reactive adhesive having self-degradation property"), a section of "Background of the Invention".

In view of the above, the inventors of present application has eagerly investigated and thereby developed a medical-use adhesive and a hydrogel resin; which satisfy general properties required for the medical-use adhesive, and is quickly disintegrated at a time a designed disintegration period is lapsed; and by which the designed disintegration period is freely controllable. Please see the WO/06/080523.

Meanwhile, there is known a group of adhesives, in which powder of "hardener" is added to liquid of "base resin", such as urea-formaldehyde resin adhesive for wood materials and novolak resin adhesive. The "hardener" is a powder of paraformaldehyde, or acid or salt for controlling pH.

Further, there has been proposed a "medical-use pressure-sensitive gluing material", in which a reaction product obtained by reacting tatratic acid with aqueous solution of N-hydroxy succinic acid is used as "hardener component" and is added to albumin solution as to induce curing of the solution. Please see JP2006-346049A ("Medical-use solid-liquid mixing type two-component adhesive being in-vivo-degradable and pressure-sensitive glueable"). There has also been proposed using of the fibrin glue in a powder form; in WO/00/038752A ("Fibrin-based glue granulate and corresponding production method"). There has been further proposed a "medical-use treatment material" in JP2005-253830A ("medical-use treatment material and its manufacturing method"); by which dextran is carboximethylated and then reacted with the N-hydroxy succinic imide to produce "carboxymethyl dextran activated with esterification", and subsequently, its powder is attached with pressing and sustained, on a sponge sheet of polyether-ester.

### Disclosure of the Invention

### Problems to be Solved by the Invention

The medical-use two-component reactive adhesive, which the inventors of present application had developed, excellent properties such as; (1) excellent adhesion performance on an adherent that is a water-containing living body; (2) relatively rapid curing and setting under normal temperature and normal pressure on surface of tissues of living body; and (3) flexibility at a level not to inhibit physical motion of the adherent while adhering on the adherent such as skins, vessels or organs until wound is healed.

*The invention is based on basic concept of above-mentioned medical-use two-component reactive adhesive and to create a totally new form of medical-use adhesive; which* enables sufficient adhesion even on wet living body portion, to which body fluid such as blood oozes out heavily, and even especially when the wet living body portion is arranged along a vertical plane.

### Means for solving the problems

Medical-use two-component reactive adhesive of the invention comprises an adhesive according to claim 1.

The epsilon-poly-L-lysine may be produced by microorganism or by enzyme.

When a kept as hydrogel, the hydrogel resin changes to sol state by self-disintegration after elapse of a period for keeping hydrogel state, which may be freely set in a range of one day to one month.

The powders of the first and second *parts* may be mixed with each other before the application, as to form a powder mixture adhesive. The powder mixture adhesive may be stored in a bottle and may be applied on the living-body portion, which is wet.

### Advantageous effect of the invention

*Obtained is a medical-use reactive adhesive having self-degradation property that* enables sufficient adhesion even on wet living body portion, to which body fluid such as blood oozes out heavily, and even especially when the wet living body portion is arranged along a vertical plane. Moreover, due to powder form, the alpha-glucan aldehyde as the first *part* may be preserved in a plastic container under room temperature up to 36 months with causing almost no drop in molecular weight. *Further, excellent obliteration of a pinhole is achieved when the application of the adhesive is made in powder form per se or is made before complete dissolving of the powder.*

### Best mode for carrying out the invention

The alpha-glucan aldehyde comprising the first part is obtained by oxidizing the alpha-glucan as to introduce aldehyde groups, and has weight-average molecular weight in a range of 1000 to 200,000. The alpha-glucan denotes sugar chains that are formed by alpha linkage of glucoses with each other. Sugar residue of glucan, which is anhydro glucose unit, has a molecular weight of 162.14. The alpha-glucan of the invention includes dextran, dextrin and pullulan, which may be used as mixed with the other. Starch or amylose may be used when properly degraded. Pullulan product of high molecular weight may also be used after being properly degraded. Introduction of the aldehyde groups may be made by typical methods of periodate oxidation. For achieving proper extent of self-degradability, number of the aldehyde groups introduced in one anhydro glucose unit is 0.1 to 1.0, more preferably 0.2 to 9.0, still more preferably 0.3 to 0.6. Curing within a sufficiently short period may be achieved even when the amount of the introduced aldehyde groups per anhydroglucose unit is 0.2 to 0.4.

Among the alpha-glucan aldehyde, dextran aldehyde and dextrin aldehyde are especially preferred because of stable adhesion performance. Weight-average molecular weight of dextran, which is used in obtaining the dextran aldehyde, is preferably 2000 to 200,000, and more preferably 2000 to 100,000. Adoptable are those commercially supplied by Pharmacosmos A/S for example; and are not only Dextran 40, Dextran 60 and Dextran 70 in medical-use grade but also Dextran T10 to Dextran T2000 among T-Dextran series. Meanwhile, dextrin product commercially supplied by Wako Pure Chemical Industries, Ltd in Japan may be used for example, in obtaining the dextrin aldehyde. Weight-average molecular weight of the dextrin is in a range of 1000 to 10,000 for example. Most preferred molecular weight of alpha-glucan aldehyde varies in accordance with detailed usage; and by selecting ones having certain molecular weight or molecular weight distribution, time length up to fluidization by self-disintegration may be adjusted. When molecular weight of the dextran aldehyde is too large, the fluidization by self-disintegration becomes excessively delayed. Meanwhile, when molecular weight of the dextran aldehyde is too small, time period for keeping the gel state becomes too short.

Weight-average molecular weight and molecular weight distribution of the alpha-glucan may be easily obtained by typical aqueous GPC (gel filtration chromatography, that is, size exclusion chromatography (SEC) in a formal naming) measurement. In detail, GPC columns formed of cross-linked water soluble polymer (TOSOH TSK gel G3000PW and G5000PW, with a TSK guard column PWH) are used as maintained at 40°C, and buffer solution (10mM KH₂PO₄+10mMK₂HPO₄) is used as eluant.

*As the powder of dextran aldehyde or the first part, one obtained as follows may be directly used as per se: the aldehyde units are introduced, subsequently freeze-dried and then mechanically pulverized. The powder might be obtained by spray drying at relatively low temperature, under depressurized condition or while being supplied with inert gas.*

Molecular weight of the polylysine may be obtained with easiness and high accuracy, by any of following methods.

### (1) SDS-PAGE (sodium dodecyl sulfate-polyacrylamide gel electrophoresis)

Measurement is easily made by use of an electrophoresis apparatus and a densitograph (AE-6920V), of Atto Corporation in Japan.

### (2) Ion association chromatography

In ion association chromatography of high-performance liquid chromatography (HPLC), reverse-phase column (TSKgel ODS-80Ts) is used for measurement. In the measurement, acetonitril is used as non-aqueous solvent while applying a gradient; and standard protein marker is used.

### (3) Aqueous GPC

Measurement may be made by use of above-mentioned aqueous GPC column for example, which is maintained at 40°C, and by use of an eluant (5% Ammonium Biphosphate/ 3% Acetonitril; pH = 4.0) that is formed by mixing of GPC-grade distilled water, phosphate buffer and acetonitril. For obtaining absolute molecular weight, low-angle laser light scattering technique may be used as combined with the GPC technique (GPC-LALLS).

The epsilon-poly-L-lysine may be produced by use of microorganism or enzyme and has molecular weight in a range of 1000 to 20,000, especially in a range of 1000 to 6000. Alpha-poly-L-lysine may also be used.

epsilon-poly-L-lysine that is obtained by following may be used, for example. Adopted strain is Streptomyces albulus subsp. Lysinopolymerus that is shown in Japan's issued patent 3525190 or 3653766. Adopted culture medium is formed of; glucose 5wt%, yeast extract 0.5wt%, ammonium sulfate 1wt%, dipotassium hydrogen phosphate 0.08wt%, potassium dihydrogen phosphate 0.136wt%, magnesium sulfate heptahydrate 0.05wt%, zinc sulfate heptahydrate 0.004wt%, and ferrous sulfate heptahydrate 0.03wt%; and is adjusted to pH6.8. After cultivation in this culture medium, the epsilon-poly-L-lysine is separated as collected from the culture medium.

When the polylysine has a too large molecular weight or excessively includes a fraction too large in molecular weight; then time period up to the fluidization by the self-disintegration becomes excessively long.

Into the second part, acid or acidic salt is added as pH adjuster. Thus, pH of mixture of the first and second parts at a time just mixed together is adjusted to a value in a range of 5.0 to 8.0, preferably in a range of 5.5 to 7.5, more preferably in a range of 6.5 to 7.5. The second part has a pH value preferably in a range of 7.0 to 9.0.

As the pH adjuster, preferably mono- or poly- carboxylic acid or anhydride thereof is added. Examples of preferred carboxylic acids are acetic acid, citric acid, succinic acid, glutaric acid, malic acid, fumaric acid and maleic acid, which are naturally occurred. These carboxylic acids have high pH adjusting ability due to buffering function and are harmless to living body. Inorganic acids or salts such as hydrochloric acid and sulfuric acid may be used alone or combined with the above-mentioned carboxylic acid, so long as the pH is adjusted to a proper value in a range of 5.0 to 8.0. Phosphate buffer solution may also be used.

Selecting either of mono-, di-, and tri- carboxylic acids as the pH adjuster enables controlling of time period up to fluidization of the cured gel, which is occurred by self-disintegration in a state of hydrogel. This is presumably due to a following reason. When polycarboxylic acid is used, quasi-cross linking is occurred between polymer chains of the polylysine or the amino-group-containing polymer, so as to delay the fluidization by the self-disintegration.

Molar ratio of aldehyde groups to amino groups, at a time the first and second parts are mixed with each other, is in a range of 0.9 to 3.5, further preferably in a range of 1.0 to 3.5. When the molar ratio of aldehyde groups to amino groups is less than such range, degradation of the produced gel would become too rapid; and when the molar ratio exceeds such range, prompt gelation would not be achievable.

*As the powder of dextran aldehyde of the first part, any form of powder may be adopted so long as the powder is excellent in dispersibility and easiness of dissolving. The form of powder is not limited so long as average particle diameter of the powder is not more than 0.1mm and the powder is easily sprayed. It is however preferred to adopt porous material having a random shaping, that is a shaping far from sphere. Thus, it is preferred to adopt freeze drying of aqueous solution and then, mechanical pulverization. Such powder is not only excellent in sprayability but also preferable for obliteration of leakage, especially for air leakage. It is considered that; such powder results in adequately inhomogeneous solution structure on course of curing, and hence, adequately inhomogeneous reaction in micrometer order; and resultantly, cured resin becomes stronger and tougher. Average particle diameter of the powder becomes more preferable step by step, as it goes from (1) to (7) in following: (1) 1-500µm, (2) 5-350µm, (3) 10-250µm, (4) 10-150µm, (5) 15-120µm, (6) 20-100µm, (7) 20-80µm. In other words, the range of 10-150µm is an especially preferred range whereas the range of 15-120µm or the like is more preferable. The average diameter of the powder may be obtained as follows; a stereomicroscopic image is taken; then a biaxial average diameter (a simple arithmetic average "x" between a long-axis dimension and a short-axis dimension) is obtained for each particle, and a length mean diameter (Σx²*/*Σx) is obtained from the biaxial average diameters, by use of an image processing program or by other method. An example of such program is Particle Size Distribution Analysis Software "Mac-View" of Mountech Co., Ltd. A mean aspect ratio (a long-axis dimension* / *a short-axis dimension) of the powder is in a range of 1.3-3.0 for example, and preferably in a range of 1.5-2.0. When the average diameter of the powder is lower than the above range and also when the average diameter of the powder exceeds the above range; dissolving of the powder at a time water is absorbed in the powder would become excessively inhomogeneous. When the average diameter of the powder is lower than the above range, "lumps" are formed on course of the dissolving; and a problem of scattering by blowing would be occurred. Meanwhile, when the freeze drying is made and then its product is pulverized with high-speed rotating blades, up to an average diameter of 10-150µm; then particles having diameters not less than 1.5 times of the average diameter is not more than 3% on a "length basis" as in the length mean diameter; and the particles having diameters not more than 1*/*2 of the average diameter is not more than 5% on the "length basis". Such a narrow-range distribution is considered to be preferable.*

*spraying out is facilitated and it is preferable in enhancement of the leakage pressure and especially in improving of an obliteration performance against an air leakage. When to store the powder mixture adhesive in a vial, its water content is kept to be not more than 2.0% and preferably not more than 1.0%. In an occasion the water content exceeds such value, molecular weight of the dextran aldehyde may be deteriorated on course of a storing period such as one year, due to hydrolysis of the dextran aldehyde.*

*the first and second parts may be sprayed to be spreaded by being blown out with compressed air. The first and second parts in a powder form may be directly sprayed on a body portion wetted by body fluid, blood or other liquid. Preferably, application procedure of spraying the powder-form adhesive is repeated, that is, made two times or more; in view of achieving evenness of spreading. At an interval(s) between and at after of such repeated application procedures, saline or distilled water is dropped or sprayed on the body portion. For example, such dropping or spraying may be made by a small syringe having a small diameter in a syringe needle or by a finger-push spray bottle for cosmetic lotion. When the body portion or an affected area, which is to be adhered to other portion, does not have enough moisture; then saline or the like is dropped or sprayed on the body portion on beforehand of initial application of the powder mixture adhesive.*

*The first and second parts may be sequentially sprayed to be spreaded on the body portion or affected area to be adhered. Nevertheless, it is preferred to prepare a powder mixture on beforehand, so that a ratio between the aldehyde group and the amino group becomes almost one; and to spray the powder mixture to be spreaded. Then, application procedure is facilitated, and varying of the mixing ratio is curbed. Thus, the powder mixture form is preferable to achieve a reliable adhesion. The powder mixture adhesive may be stored for a long period at room temperature, in an air-tightly closed bottle or in a container having a desiccant. In place of spraying with compressed air or gas for the application of the powder mixture adhesive, it may be instantly dispersed in water and sprayed with compressed water.*

The first and second parts may be easily sterilized by irradiation with radioactive rays, preferably by irradiation with 10 to 50Kgy of electron beams and more preferably by irradiation with 20 to 30Kgy of electron beams. Conditions of the irradiation sterilization are set in a manner that no adverse effect is incurred on performance of the adhesive such as cure time.

Mixing of the first and second parts and application may be in various ways when to use the two-component reactive adhesive of the invention.

When the first and second parts are mixed with each other, aldehyde groups on the alpha-glucan aldehyde are reacted with amino groups on the amino-groups-containing polymer as to form Schiff linkages, which makes cross linkages so as to form hydrogel having network structure of the polymer chains. Resultantly, curing is made within 2 to 150 seconds, preferably within 3 to 100 seconds, and more preferably within 5 to 50 seconds, from the mixing. Preferred time period up to the curing from the mixing varies to a certain extent in accordance with usages, and is no less than 10 seconds, no less than 15 seconds in particular, when to penetrate into inside of living tissues to exhibit high level of adhesion strength.

Cured adhesive layer or resin in a state of hydrogel, which is formed by curing reaction, changes into fluid state by self-disintegration when a designed fluidization period has elapsed. In other words, the cured adhesive layer or the resin, if being kept as a hydrogel, changes into a fluidized state, which is a flowable sol state, even without undergoing enzymatic degradation. Therefore, the cured adhesive layer or the resin, when being placed in living body, disappears after elapse of a predetermined time period, by being absorbed or excreted. The designed disintegration time period may be arbitrarily set in a rage of a few hours to 4 months, typically in a range of one day to one month, especially in a range of two days to two weeks.

On contrary, conventional biodegradable resins, which are degraded and absorbed in living body only through enzymatic degradation, vary in respect of time period for the degradation. It has been difficult to design the conventional biodegradable resins so as to be rapidly degraded after elapse of a required time period for keeping the adhesion strength.

The time period for self-disintegration may be arbitrarily controlled and set; by selection or controlling of molecular weights of, and molecular-weight distribution, of the alpha-glucan aldehyde and/or the poly-L-Lysine; by use or non-use and selection of polycarboxylic acids; and by controlling of pH at a time the first and second liquids are mixed. Thus, it is able to arbitrarily design the time period for being disintegrated and absorbed, by controlling of compositions of the two-component adhesive.

While mechanism of the self-disintegration is not clear, we consider that; alpha-glucoside linkage adjacent to the Schiff linkage has become easy to be cleaved where aldehyde groups of the alpha-glucan aldehyde have reacted with the amino groups.

The medical-use adhesive and medical-use resin of the invention may be preferably applied for bonding in the living body, filling between tissues, haemostasis, embolization of vessels, sealing of aneurysm, and substrate for drug delivery system (DDS).

### Embodiment

### <Powder-liquid Medical Adhesive> Reference example

### A1. Preparation of powder dextran aldehyde (the first part)

Twenty grams of dextran (Wako Pure Chemical Industries, Ltd; Lot No.EWN0778) having weight-average molecular weight of 200,000 was dissolved in 100ml of distilled water. Then, various amount of sodium periodate (MW 213.89) was added and stirred at 40°C for 5 hours as to proceed reaction. After the reaction, solution was subjected to dialysis for 24 hours by use of distilled water and dialysis membrane of fractioning molecular weight of 14,000, and thereafter being freeze dried. Subsequently, dried product was subjected to pulverization for 1 minute by use of a portable crusher ("Wonder Blender WB-1" of OSAKA CHEMICAL Co., Ltd.), as to obtain the dextran aldehyde in a form of powder.

The amount of the introduced aldehyde groups per anhydroglucose unit (mole) was 0.28. The amount of the introduced aldehyde groups was measured by redox titration method. In detail, 20mL of 0.05mol/L iodine aqueous solution, 10mL of 10mg/mL dextran aldehyde solution and 20mL of 1mol/L sodium hydroxide aqueous solution were charged into a 100mL Erlenmeyer flask, and stirred at 25°C for 15 minutes. Then, the solution was added with 15ml of 6v/v% sulfuric acid aqueous solution, and was titrated with 0.1 mol/L sodium thiosulfate aqueous solution. Time point at which reaction system becomes colorless and transparent was deemed as ending point, while starch aqueous solution was used as indicator.

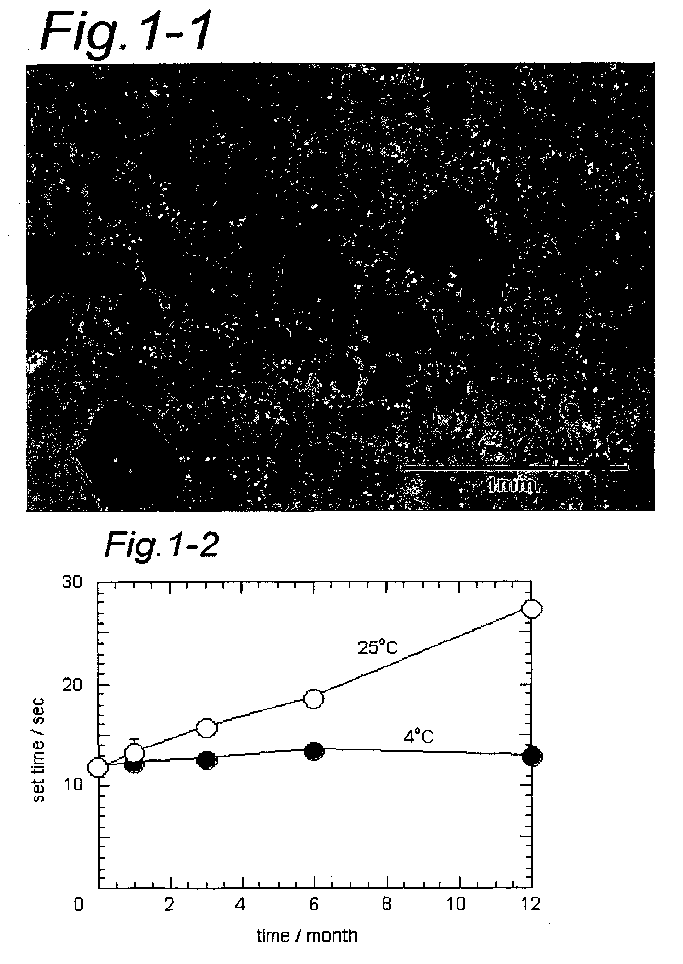

Particle sizes of the powder were evaluated by stereomicroscope as to reveal that; the powder has an average particle diameter of about 90 micrometer as shown in a photograph of Fig. 1. Moreover, electron microscope observation on surface of the powder revealed porous nature of the powder.

### A2. Preparation of epsilon-polylysine aqueous solution (the second part)

Then, 25wt% polylysine aqueous solution (molecular weight of 4000; Chisso Corporation, Lot No.2050506, free amine) was added by acetic anhydride and distilled water, in a manner that concentration of the acetic anhydride becomes 2wt%, as to prepare 10wt% polylysine neutral aqueous solution.
(All the remaining part of the description of previous PCT application JP2006-301543 is discarded, except for second paragraph of section "16. An example of use in respiratory surgery Obturation of air leakage from lung")

### A3. Preservation stability of the dextran aldehyde

The powder dextran aldehyde obtained in Section A1 was put in polyethylene bottles and kept respectively at -20°C and at +50°C. At an elapse of 40days, the two samples of the powder were subjected to molecular weight analysis by use of GPC device; and peak tops of obtained curves indicated a molecular weight of 19,223 for a sample having been kept at -20°C and a molecular weight of 18,659 for a sample having been kept at +50°C. In other word, no deterioration due to high temperature storage was observed. Meanwhile, the dextran aldehyde same as above was dissolved to prepare 20wt% aqueous solution, which was then kept at +50°C; and molecular weight analysis as in the above reveals that 14days and 28 days storage caused drops of molecular weight respectively to become 84% and 71% of the original one.

On the other hand, each of the aqueous solutions of the dextran aldehyde was mixed with the 10wt% polylysine neutral aqueous solution obtained at Section A2; and curing (gelling) time of the mixture was measured as to evaluate extent of deterioration of the dextran aldehyde. *The gelling time was measured as follows. Firstly, 0.5mL of the first liquid, which is 20wt% aqueous solution of the dextran aldehyde powder of Section A1, was taken* into glass test tube having diameter of 16mm, into which magnetic stirrer bar having a diameter of 4mm and a length of 10mm was putted, and was heated to 37°C and stirred at a rate of 100rpm. And, 0.5mL of the second liquid of Section A2, which had been preheated to 37°C, was added to the solution by micropipette. Time length up to a timepoint at which the stirrer bar ceased its rotating as a result of curing was measured by use of a stopwatch. Table 1 shows change of *gelling time when the solution of dextran aldehyde was kept at +50°C for two weeks and for four weeks.* *Fig.1A* *shows changes of gelling time on course of storing the solution of dextran aldehyde at +25°C up to one year as well as those at +4°C for same period. For experiments for* *Fig. 1A**, succinic anhydride has been added to the polylysine solution in place of acetic anhydride.*

**Table 1. Change in property of dextran aldehyde kept in an aqueous solution, at 50°C (as an acceleration test) ¹⁾**

| Time / week | Gelling time / second ²⁾ | Relative molecular weight | Relative aldehyde amount |
|---|---|---|---|
| 0 | 10.2±0.0 | 100 | 100 |
| 2 | 24.7±0.4 | 84 | 95 |
| 4 | 50.5±1.6 | 71 | 89 |

| | | | |
|---|---|---|---|
| 1)ald-dextran, 75K, -CHO=0.43/sugar unit, 20 w/w% 2)with 10%poly(L-lysine)(containing 2% acetic anhydride), data = mean±s.d.(n=3)) | | | |

Results *of change of the gelling time shown in Table 1 and* *Fig.1A* *indicate* that elapse of 14days and 28 days at +50°C respectively correspond to 1 year and 3 years at 25°C. *This suggests that, when the dextran aldehyde is stored at 25°C in a powder form and not in an aqueous solution, molecular weight of the dextran aldehyde would not be changed for 3 years or more. Moreover, as shown in* *Fig.1A**, when aqueous solution of the dextran aldehyde was stored at 4°C, no significant change in the gelling time was observed even after one year. This indicates that long time storing of the dextran aldehyde in a form of aqueous solution would be feasible.*

### A4. Sealing performance

The powder dextran aldehyde obtained at Section A1 and the neutral polylysine aqueous solution obtained at Section A2 were used to form a hydrogel and to evaluate sealing performance of the hydrogel. Details of procedures were as follows.

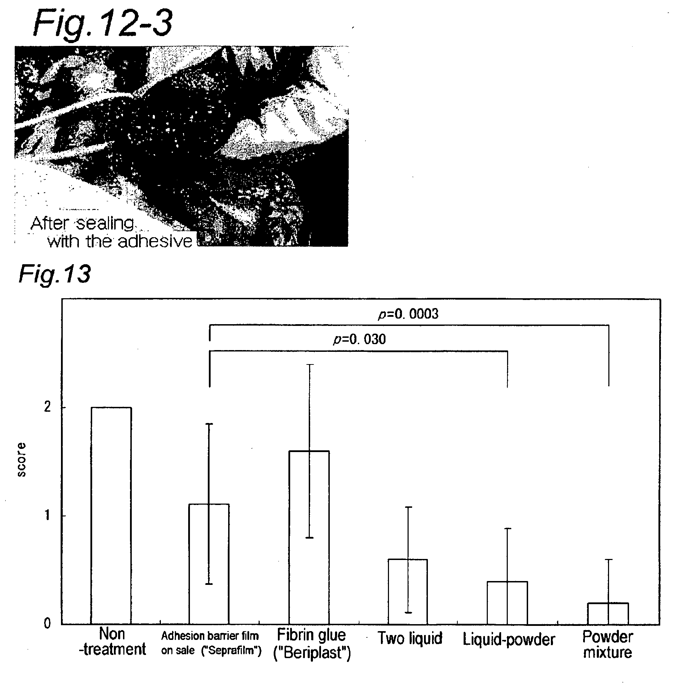

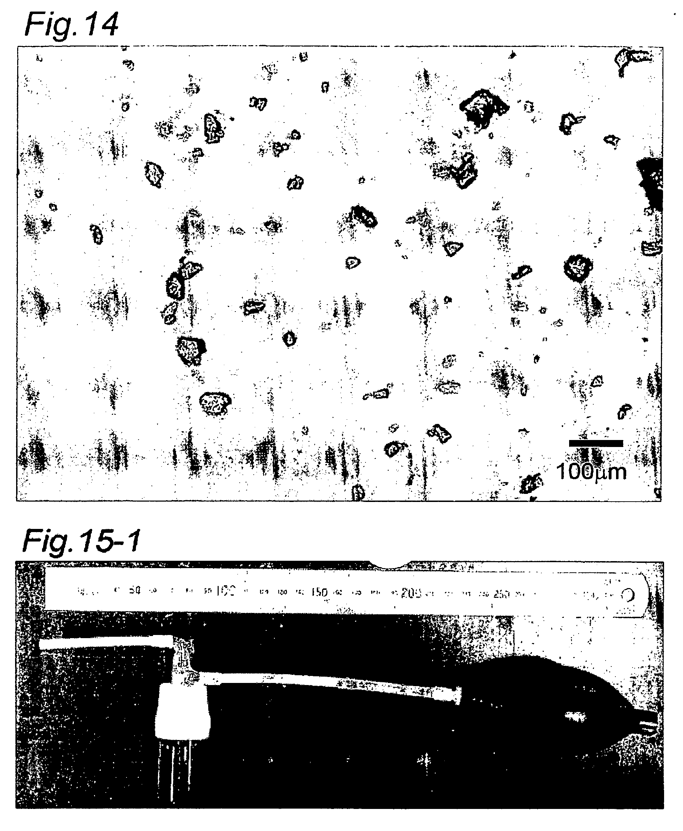

Firstly, a hand-made powder-spraying device 10 was prepared by use of a dropper cap, as shown in a photograph of Fig. 13 and a schematic illustration of the device, of Fig. 14. In detail, a main Pyrex glass tube 11 having outer diameter of 5mm and inner diameter of 3mm was bent by heating almost perpendicularly; a silicone tube 2 having outer diameter of 6mm and inner diameter of 4mm was attached on an end of the main Pyrex glass tube 11; and an outer Pyrex glass tube 12 having outer diameter of 10mm and inner diameter of 8mm was fusion bonded on a portion of the main Pyrex glass tube 11, as to form a glass tube fixture 1. A silicone-rubber dropper bulb 3 having a 10ml volume was charged with about 3g of the dextran aldehyde powder 4 and then securely attached on the outer Pyrex glass tube 12 of the glass tube fixture 1.

A cap formed of polyethylene and having diameter of 37mm and height of 20mm, of a bottle for chemicals, was perforated at its center to form a hole in a diameter of 10mm, and then placed on a laboratory bench in a manner that inner face of the cap was exposed upward. Then, 0.5g of the powder-form first part of the adhesive was thinly and almost evenly sprayed on the inner face of the cap, by use of the powder-spraying device 10. Subsequently, 0.1g of the liquid-form second part of the adhesive was sprayed thereon; and the 0.5g of the powder-form first part of the adhesive was thereon applied by use of the powder-spray device. Obtained gel was gelatinous and fully flexible. Two minutes later, the cap was screwed to be fixed on main part of the bottle for chemicals, which is formed of polyethylene and has a volume of 500mL and a height of 175mm. On beforehand of the screwing, bottom part of the bottle had been cut out. The bottle having been screwed with the cap was overturned and water was slowly poured into a barrel part of the bottle. Resultantly, pouring of 500g water did not cause rupture of the gel and leakage of water. Thus, high level of sealing or water-tight performance was confirmed.

### A5. Adhesiveness and flexibility

Adhesiveness on a tissue and flexibility was evaluated for the adhesive resin using the powder dextran aldehyde obtained in Section A1 and the neutral polylysine solution obtained in Section A2. In detail, a beagle dog under anesthesia was intubated and its lung was exposed while motion of the lung was maintained with artificial respirator. On surface of the lung, 1g of the powder-form first part of the adhesive was thinly and almost evenly sprayed by use of the powder spray device used in Section A4. Then, 0.5g of the liquid-form second part of the adhesive was almost evenly dropped thereon. Further thereon, 1g of the powder-form first part was sprayed in same manner as above.

Two minutes later, a pressure was applied to the lung by supplying oxygen through the airway (respiratory tract). And, it was examined whether the gel was peeled off from the lung surface. Resultantly, no peeling off from the lung surface was observed even under pressure of 40cmH₂O or more. Moreover, repeating of inflation and deflation of the lung did not cause rupture of the gel, as to confirm a high level of flexibility of the gel.

### A6. Haemostasis on liver

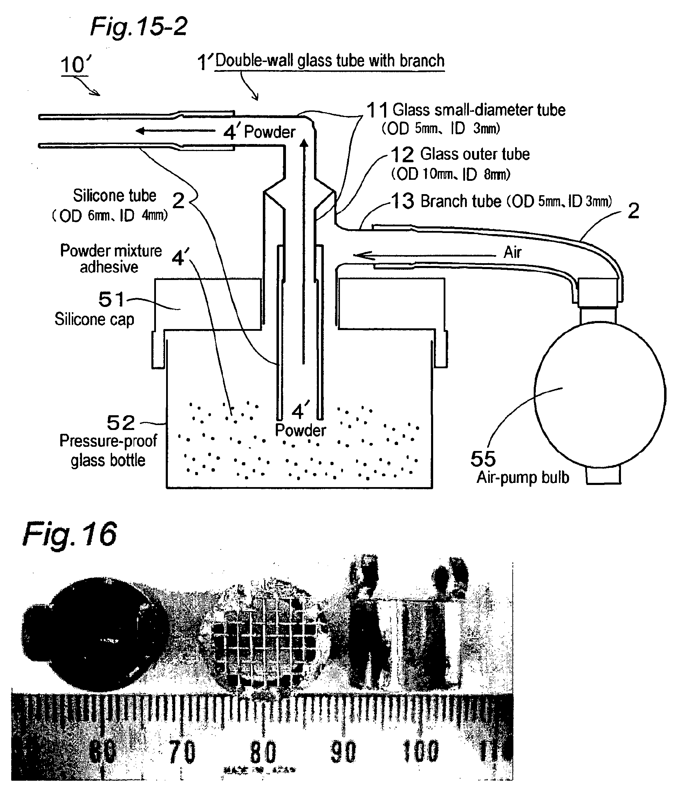

Oozing of blood from a partially cut out portion of the liver was arrested by use of the adhesive formed of the powder dextran aldehyde of Section A1 and the neutral polylysine solution of Section A2, as follows in detail. After an artery in the liver was ligated, the liver was partially cut out by use of a forceps, as to expose a cut-out surface. On course of such cutting, blood vessels were cut off by use of an electric scalpel; and cutting off of each blood vessel having diameter more than 1mm was made only after its litigation. The cut-out surface 5 arranged substantially vertical, which is an increscent half-moon or first-quarter-moon area on center of photograph of Fig. 15, was thinly and almost evenly sprayed with 2g of the powder-form first part of the adhesive by use of the powder spray device in Section A4. Subsequently, 1g of the liquid-form second part of the adhesive was almost evenly sprayed thereon, by use of a fibrin glue spray device ("Bolheal spray set" of TEIJIN PHARMA LIMITED), which is a double-syringe and air-driven spray device. Further, 2g of the powder-form first part of the adhesive was sprayed on the cut-out surface.

Two minutes later, the cut-out surface 5 having been arrested of bleeding was applied with a gauze pad, weight of which had been measured on beforehand, and then, litigation of the artery was undone so that bleeding amount was obtained by measuring of the gauze pad soaked with blood. Resultantly, only 1g of bleeding was observed for a period of 2 minutes directly after undoing of litigation, as to exhibit a high level of haemostasis effect as indicated in Fig. 15. Meanwhile, when the cut-out surface 5 was untreated, 10g or more bleeding was observed for same period and under same conditions.

### A7. Tissue Antiadhesion

An experiment of tissue antiadhesion was made by the adhesive resin using the powder dextran aldehyde obtained in Section A1 and the neutral polylysine solution obtained in Section A2. An SD (Sprague-Dawley) rat having 300g of body mass was subjected to midline incision; and then, left-hand-side peritoneum and a portion of muscle layers were removed in a square-shaped area having about 2.5cm on each side, with a depth of about 1mm. An electronic scalpel was applied to whole of the square-shaped area. Subsequently, 1g of the powder-form first part of the adhesive was thinly and almost evenly sprayed on the square-shaped area by use of the hand-made device of Section A4; and thereon, 0.3mL of the liquid-form second part of the adhesive was thinly and almost evenly applied by dropping. Further thereon, 1g of the powder-form first part was sprayed by use of the hand-made device. Then, two minutes later, abdominal cavity was closed.

Two weeks later, the abdominal cavity was opened and the extent of tissue adhesion between the peritoneum and a greater omentum was graded by a following three-grade scoring; "0" for no adhesion, "1" for peelable and mild adhesion and "2" for non-peelable and strong adhesion. For comparison, an adhesion barrier membrane (Seprafilm^{™} of Genzyme Corporation) having been on sale was attached on the square-shaped area on each of the rats in a comparison group. Obtained scoring of tissue adhesion was; 2.0 ± 0.0 for a group of rats with no anti-adhesion membrane, 1.1 ± 0.7 for "Seprafilm" group, and 0.4 ± 0.5 for a group of rats treated with the adhesive mentioned on top of this section. Probabilities of non-existence of significant difference from the non-treatment group are as follows; P=0.02 for the "Seprafilm" group and P=0.0001 for the group of the adhesive of the embodiment. Number of samples in each group was; 6 for the non-treatment group, 9 for the "Seprafilm" group and 10 for the embodiment group.

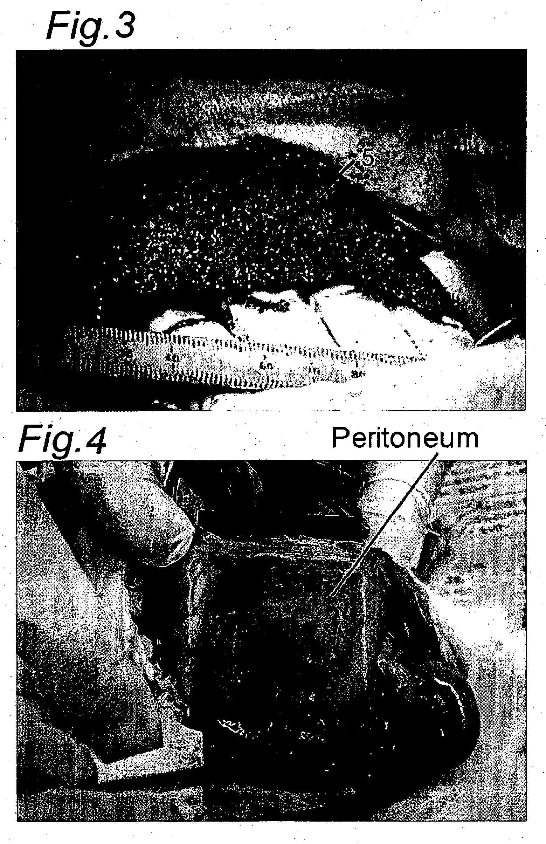

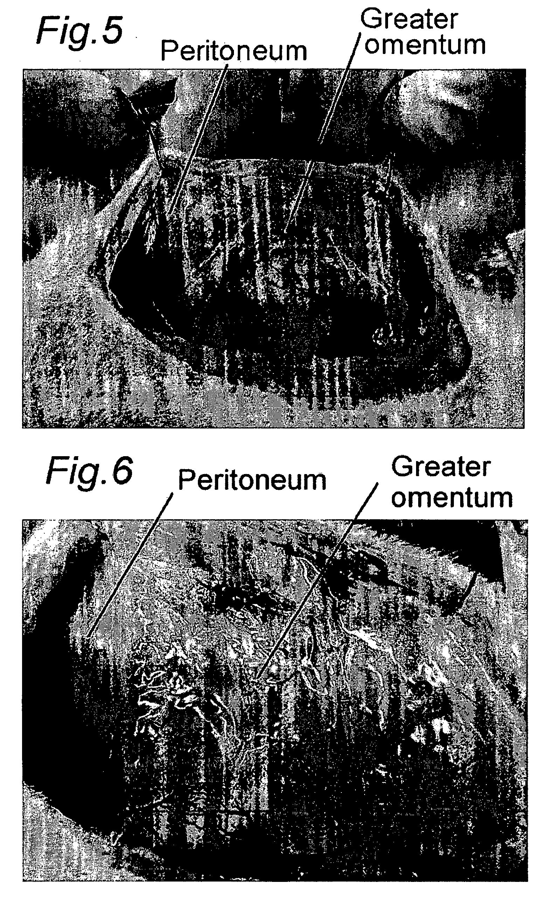

As clear from photographs of Figs. *4-6*, the Seprafilm group (Fig. *5*) and the embodiment group (Fig. *4*) showed significant tissue anti-adhesion effect compared to the non-treatment group (Fig. *6*); and the embodiment group (Fig. *4*) showed a significantly higher tissue anti-adhesion effect than the Seprafilm group (Fig. *5*), with probabilities of non-existence of significant difference being 0.03. As shown in an example of Fig. *4* for the embodiment, tissue adhesion between the peritoneum and the greater omentum was mild even when being existed. On contrary, example of Fig. *5* for the Seprafilm group showed a tissue adhesion in a large area.

**Table 2 Tissue Anti-adhesion effect**

| | Tissue adhesion extent | Non-significancy Probability from Non-treatment | Non-significancy Probability from Seprafilm |
|---|---|---|---|
| Non-treatment | 2.0 ± 0.0 | | |
| Seprafilm | 1.1 ± 0.7 | P=0.02 | |
| Embodiment | 0.4 ± 0.5 | P=0.0001 | P=0.03 |

### <Two-Powder Medical Adhesive >

### B1. Powder dextran aldehyde (the first part) and powder polylysine (the second part)

"Dextran 70 powder J.P., Pharmaceutical grade (for injections)" of Meito Sangyo Co., Ltd. was used. Except for this, the powder dextran aldehyde was obtained in same manner as described in Section A1, and was used as the first part of the adhesive in following experiments. The amount of the introduced aldehyde groups per anhydroglucose unit (mole), which was measured in same manner as in Section A1, was 0.28 as same as in Section A1. Stereomicroscopic evaluation as same as in Section A1 revealed that; the powder has an average particle diameter of about 90 micrometer. Similarly, the powder was revealed to have porous nature. *Further, average aspect ratio, or average ratio of long axis diameter to short axis diameter, was about 1. 6.*

Meanwhile, a *10wt% neutral polylysine aqueous solution was freeze dried and then mechanically pulverized to* obtain a powder, *in same manner with above-mentioned way of obtaining the powder dextran aldehyde, and was used as the second part of the adhesive. The 10wt% neutral polylysine aqueous solution used here was obtained as follows; 25wt% polylysine aqueous solution (molecular weight of 4000; Chisso Corporation, Lot No.2050506, free amine) was added by 0.5 g of succinic anhydride and 14.5g of distilled water. The stereomicroscopic evaluation was made for thus obtained powder polylysine, in same manner as made for the powder dextran.* When the powder dextran aldehyde and the powder polylysine, in this Section B1, were mixed in weight ratio of 4/1, molar ratio of aldehyde groups and the amino groups becomes almost 1.

In following experiments, used was such a powder mixture adhesive, in which the powder-form first and second parts of the adhesive are mixed with each other, with the molar ratio of almost 1. *Such powder mixture adhesive is stored in a glass vial having aluminum cap as to keep water content of the powder mixture adhesive not more than 1.0%.*

### B2. Use in respiratory surgery Obturation of air leakage from lung 1

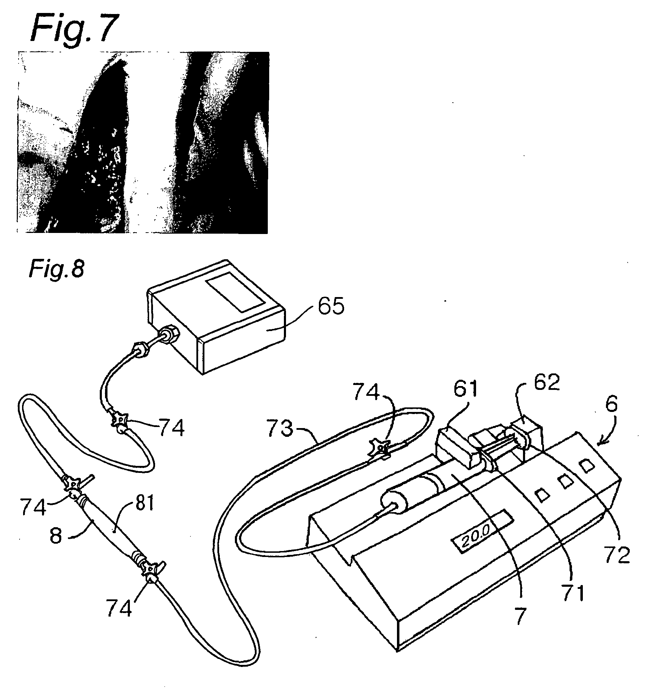

To confirm obturation of the air leakage from lung, following experiment was made by use of a beagle dog. In accordance with common procedures, conducted were anesthesia, endotracheal intubation, and then opening of thorax; and a *circular area having diameter of 15mm* was cut out on right lung by use of an electrical scalpel, *to form a pleural deficient area*, which is a dark color part at near center of Fig. *7* and slightly leftwardly deviated from the center. Physiological saline was sprayed on the deficient area, and pressure of respirator was increased, as to confirm occurrence of air leakage. Subsequently, the two-part adhesive was applied; and two minutes later, thorax was filled with physiological saline and a leakage test was made. As a comparative adhesive, the fibrin glue ("Bolheal", KAKETSUKEN in Japan) was used and fibrinogen solution was rubbed to be spreaded on the deficient area by fingers and then further applied thereon by use of a sprayer kit (rub & spray method). In regard of using of fibrin glue, leakage test was made 5 minutes after the application, in accordance with science literature(s).

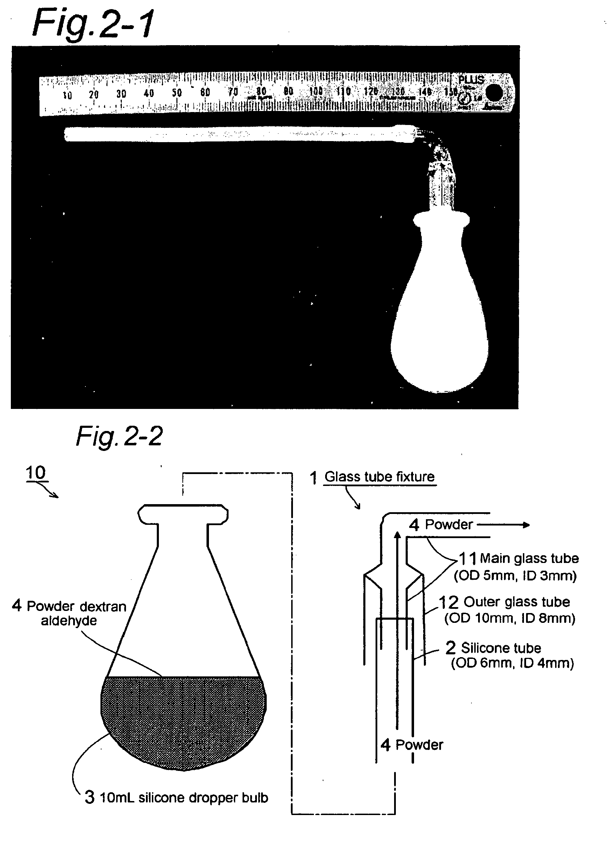

Spreading of the adhesive was made as follows. Firstly, about 0.5mL of physiologic saline was evenly dropped on the plural deficient area and its surroundings by use of 1 mL volume syringe. Thereon, 0.2g of the powder mixture adhesive was almost evenly and thinly sprayed by use of the hand-made spray device *(**Figs. 2-1* *to 2-2)*. Further thereon, about 0.5mL of physiological saline was again dropped. Fig. 7 is a photograph showing the pleural deficient area that is obturated by the above procedures. As seen from the Fig. 7, the obturation is achieved by hydrogel resin.

Two minutes later, a pressure applied through the air way (respiratory tract) by an artificial respirator was increased gradually from 5cmH₂O. Then, air leakage was observed at a pressure *about 30*cmH₂O. Thus, even by such a simple experiment, the adhesive of the embodiment was confirmed to effect obturation of air leakage at least in same level with the fibrin glue.

### B3. Obliteration of a pinhole on an artificial blood vessel

An artificial blood vessel (Gore-Tex^{™}, diameter of 8mm) was perforated to have a pinhole of 18G (outer diameter of 1.3mm for circular area). Then, the powder mixture adhesive was sprayed to be applied there. In detail, on vicinity (area of 1cm²) of the pinhole, dropping of distilled water by the 1mL syringe and applying of the powder mixture adhesive (aldehyde group/amino group molar ratio = 1) by use of the hand-made spray device *(**Figs. 2-1 to 2-2**)* were made as in following sequence; #1--dropping of distilled water, #2--spraying of the powder mixture adhesive, #3--dropping of distilled water, #4--spraying of the powder mixture adhesive, and #5--dropping of distilled water. Spread of the powder mixture adhesive as a sum of two times of application was about 0.2g/cm². *For sake of visibility, the water had been added by "Brilliant Blue FCF" of (Wako Pure Chemical Industries, Ltd; Lot No.KLN3789)*

Two minutes later, hydraulic pressure was applied with water from one end of the blood vessel and effect of preventing of water leakage through the pinhole was evaluated. *In detail, a syringe pump device 6 (TERUFUSION Syringe Pump STC-523 of Terumo Corporation) as shown in* *Fig.8* *was used to pump up the blood vessel 8 with saline, at a rate of 20mL*/*h, so as to gradually increase hydraulic pressure in the blood vessel. As shown in* *Fig. 8**, a plastic syringe 7 of 30mL capacity has been attached onto the syringe pump device 6, where a stationary arm 61 and a movable arm 62 of the syringe pump device 6 are abutted respectively on front face of finger rest 7 and on rear face of a piston rod, of the syringe 7. Saline is pumped out by frontward shifting of the movable arm 62 and flows through a silicone tube 73 having inner diameter of 8mm and tube joints 74, each of which has a stopper cock formed of fluorine resin and which are arranged at a distal end and around halfway position, of the silicone tube 73; and then enter the blood vessel at its one end. The tube joint 74 at the distal end of the silicone tube 73 is a three-way stopcock as to enable release of accumulated pressure after completing of measurement. One of branch tubes of the three-way stopcock is inserted to the one end of the blood vessel 8. At around this one of the branch tubes, a Nylon cord is tightly wound, to prevent leakage along joined faces.*

*Leakage of dyed saline through the pin hole 81 of the artificial vessel 8 was monitored. And, a pressure value at a time the leakage was firstly observed was read out from a pressure monitor 65, and was taken as a leakage pressure.*

*For sake of comparison, fibrin glue ("Beriplast P Combi-Set" of CSL Behring Co. Ltd.) was applied on an area of the pin hole, in accordance with a prescribed method, in a manner that spread of each of A and B liquids becomes 1mL*/*10cm² ; by which, the A liquid was rubbed to be spread on first hand, and then A and B liquids were mixed and sprayed, by use of a commercially-supplied spray device ("Bolheal spray set"); "Rub & Spray method";* Naoki Minato et al "New Application Method of Fibrin Glue for More Effective Haemostasis in Cardiovascular Surgery" Jpn. J. Thorac. Caridiovasc. Surg. 2004; 52: 361-366*.*

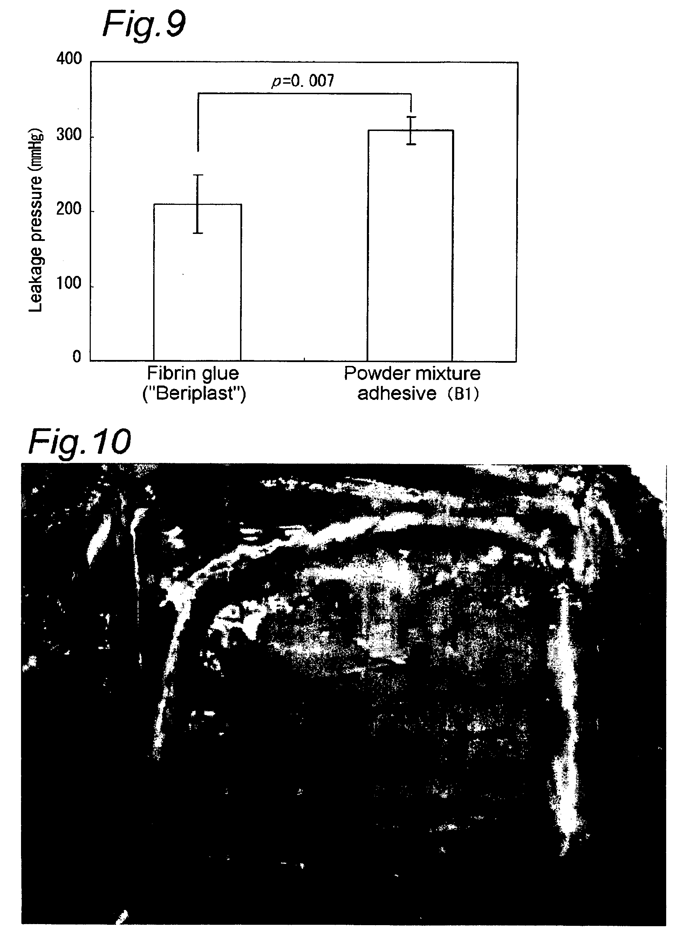

*Four times of experimental runs were made each of; the powder mixture adhesive of the embodiment and the fibrin glue; and obtained results are shown in Table 3 and* *Fig.9**. The leakage pressure when the fibrin glue is adopted was in a range of 167-262mmHg. And, on contrary, when the powder mixture adhesive of the embodiment was used, the leakage pressure was over 320mmHg for three times of the experimental runs among the four times of runs, and was 277 mmHg for only one time of the experimental run. The "over 320mmHg" means exceeding of a limit of measurement because of occurrence of leakage other than the pin hole or the like. Therefore, the adhesive of the embodiment exhibit a remarkably excellent performance on obliteration of the pinhole compared with the fibrin glue; test of significance revealed p=0.007.*

**Table 3**

| | 1 | 2 | 3 | 4 | Average ± SD |
|---|---|---|---|---|---|
| Fibrin glue | 232 | 262 | 181 | 167 | 210±18 mmHg |
| Powder mixture adhesive | >320 | 277 | >320 | >320 | 309±6 |

### B4. Degradability in abdominal cavity

A rat was anesthetized and subjected to midline incision; and then, right-hand-side peritoneum and a portion of muscle layers were removed in a square shape having about 2.5cm on each side, with a depth of about 1 mm, to form a peritoneum deficient area. On the peritoneum deficient area, spraying of physiological saline by a spray bottle and applying of the applying of the powder mixture adhesive (aldehyde group/amino group molar ratio = 1) *of B1 section* by use of the hand-made spray device *(**Figs. 2-1 to 2-2**)* were made as in following sequence; #1--dropping of physiological saline, #2--spraying of the powder mixture adhesive, #3--dropping of physiological saline. *Spread of the powder mixture adhesive was about 0.1g*/*cm²;* and spread of the physiological saline was 0.1mL/cm² at each spraying.

One week later, the abdomen cavity was opened and remnant of the hydrogel resin layer of the adhesive was observed by naked eye, as to reveal that *almost all* the hydrogel resin layer was *disintegrated and disappeared* as shown in Fig.*10**.* When the two-liquid medical adhesive of the Example 1 on Section 7 of *WO*/*06*/*080523* was used in place of the adhesive of this section, about 90% or more of the hydrogel resin layer was disappeared on elapse of one week. Hence, disintegration rate of the two-powder medical adhesive was observed to be smaller than that of the two-liquid adhesive.

*Meanwhile, the powder mixture adhesive same as the above was twice spreaded on the peritoneum deficient area so that total spread becomes 0.2g*/*cm²; and then, as in the above, the abdomen cavity was opened and remnant of the hydrogel resin layer of the adhesive was observed by naked eye*, as to reveal that about 50% of the hydrogel resin layer was remained as shown in Fig.*10*. In a photograph image of the Fig. 20, the hydrogel resin layer covers a circular exposed area, which is arranged at near center as slightly shifted upward from the center. *Hence, when the spread of the adhesive was doubled, disintegration rate of the resin in the abdomen cavity became smaller.* Therefore, it was revealed that; the two-powder medical adhesive is applicable to an occasion requiring 1-2 weeks or more of retention period, such as haemostasis on liver, *merely by increasing of the spread*, even without addition of polycarboxylic acids as in Section 8 *of* WO/06/080523*,* and without addition of high-molecular-weight amine-groups containing polymer as in Section 6 *of* WO/06/080523.

Haemostasis performance of the two-powder adhesive was revealed to be superior to the two-liquid adhesive of WO/06/080523 *(especially to its* Examples 1 and 2), because the *powder-mixture* adhesive is more excellent in absorbing moisture in the tissues. Thus, the *powder-mixture* adhesive was observed to be capable of sufficiently arresting blood oozing or similar extent of bleeding.

### B5. In vivo disintegration after obliteration of air leakage from lung of beagle dog

*A circular pleural deficient area having diameter of 15mm was formed on right lung of a beagle dog. In detail, a circular silicone sheet having diameter of 30mm, on which a circular hole having diameter of 15mm had been opened on beforehand, was pasted on the lung; cyanoacrylate resin liquid was poured into the circular hole; then, one minute later, the silicone sheet wad detached; and pleural membrane at an area of the cyanoacrylate resin was cut out by a scalpel.* On the peritoneum deficient area, *the powder mixture adhesive of Section B 1 was applied in a manner same as in Section B 1. Moreover, effect of twice spreading and thrice spreading of the adhesive were also investigated. A procedure for the twice spreading of the adhesive was made in* following sequence; #1--dropping of physiological saline, #2--spraying of the powder mixture adhesive, #3--dropping of physiological saline, #4--spraying of the powder mixture adhesive, and #5--dropping of physiological saline. *A procedure for the thrice spreading of the adhesive was made in same manner as the above. Thus, total spread of the adhesive was about* 0.2g/cm² *for the twice spreading, and was about 0.3g*/*cm² for the thrice spreading.*

*One week and two weeks later, the thorax cavity was opened and remnant of the hydrogel resin layer of the adhesive was observed by naked eye, as to reveal that; a slight portion of the hydrogel resin layer was remained at after one week; and almost all the hydrogel resin layer was disintegrated and disappeared at after two weeks.*

*Results of the twice spreading and the thrice spreading of the adhesive showed no significant difference with the result of one-time spreading when observed with naked eye. Nevertheless, detailed observation on tissue section revealed that amount of remaining hydrogel increased with increase of time of spreading the adhesive.*

### B6. Haemostasis in cutting out part of kidney

A rabbit was used to explore whether it is capable to arrest bleeding from a cut-out face that is formed by cutting out of a portion of the kidney. In same manner as in Section A6, in which the two-liquid adhesive was used in arresting of bleeding of liver, arteries in the kidney were clamped and partially cut out, and bleeding was arrested by the two-powder adhesive. Figs. 21-1 to 21-3 are photographs showing these procedures; Fig. 21-1 shows the kidney to be cut out; Fig. 21-2 shows the kidney right after the cutting; and Fig. 21-3 shows the cut-out face having been applied with the adhesive. Application of the two-powder adhesive was made in same manner as in Section B2, as follows. On the cut-out face of about 5cm² area, repeated twice were; dropping of 1 mL of physiological saline by 1 mL syringe and thereafter spraying of 0.5g of the powder mixture adhesive. And, finally, 1mL of physiological saline was dropped there.

Two minutes later, the clamping was released and bleeding amount was evaluated by measuring weight of a gauze pad before and after absorbing of blood bled out from the cut-out face. Total amount of bleeding for 10 minutes period was measured as 0.3g for a group treated with the two-powder adhesive and 20g for a non-treated group. Thus, the two-powder medical adhesive of the embodiments is expected to have sufficient haemostasis performance when for partially cutting out of kidney.

### B7. Tissue Antiadhesion

*A tissue antiadhesion effect of the above powder mixture adhesive of Section B1 was observed by use of SD (Sprague-Dawley) rats having 300-310g of body mass, by a method as in Section A7 except that; here, affected area was washed with Ringer's solution (NaCL 0.9%, KCL 0.3%, CaCL₂ 0.2%) after removing of the peritoneum and applying of the electronic scalpel. Then, the affected area was sequentially subjected to; #1--dropping of the Ringer's solution, #2--spraying of the powder mixture adhesive, and #3--dropping of distilled water, so that total spread of the adhesive became about 0.1g*/*cm².*

*Evaluation of the results was made as in Section A 7 as follows.* Two weeks later, the abdominal cavity was opened and the extent of tissue adhesion was graded by *the* three-grade scoring. For comparison, following four groups were adopted: #1--the adhesion barrier membrane ("Seprafilm') having been on sale was attached on the *affected area; #2--the fibrin glue ("Beriplast P Combi-Set") was applied on the affected area as in Section B3, #3--20% aqueous solution of the powder dextran aldehyde of Section A1 was mixed with same weight of the polylysine aqueous solution of Section A2, and obtained mixture was applied on the affected area so that spread on basis of solid content of the mixture solution became 0.1g*/*cm²; and #4--the powder-liquid two-part reactive adhesive same as described in Section A4 was applied on the affected area, by a method same with that in Section A4.*

*Results of these are shown in* *Fig. 13**. As shown in* *Fig.13**, excellent antiadhesion effect was achieved by the powder mixture adhesive and the powder-liquid adhesive of the embodiment, in a manner exactly same with that by the two-liquid reactive adhesive of WO*/*06*/*080523. Achieved antiadhesion effect was significantly higher than that by the commercially available adhesion barrier membrane. As also shown in* *Fig.13**, the anti-adhesion effect seemed to be largest by the powder mixture adhesive. Probabilities of non-existence of significant difference from the group of the commercially available "Seprafilm" are as follows; P=0.0003 for the group of the powder mixture adhesive and P=0.0030 for the group of the powder-liquid adhesive.*

*When the powder mixture adhesive of the embodiment was adopted, a slight remnant of the hydrogel resin layer of the adhesive was observed by naked eye, even when the abdomen cavity was opened two weeks later. Reason why the antiadhesion effect of the powder mixture adhesive appeared to be slightly higher might be that the hydrogel resin layer lasted for longer time than the other groups.*

### B8. Powder mixture adhesive having smaller particle size, and its spreading

*The powder mixture adhesive in same manner as in Section B1 is prepared except that; condition of mechanical pulverization was changed to decrease average diameter of the obtained powder. The fine pulverizer ("Wonder Blender WB-1" of OSAKA CHEMICAL Co., Ltd.*) *used in Section B1 was also used; and duration for pulverization is increased from 10 second, which was adopted for Section B1, to 30 seconds.* *Fig.14* *shows a stereomicroscopic image of thus obtained powder mixture adhesive. Particles larger than 50 micrometers are almost not found and are considered to be not more than 1%. The average particle diameter of thus obtained powder mixture adhesive was about 30 micrometers. This powder mixture adhesive was used only in Sections B9 and B10.*

*Meanwhile, in place of the powder-spraying device using the dropper bulb shown in* *Figs.2-1 and 2-2**, adopted is a spray device that is comprised of a pressure-proof bottle 52 storing the powder of the adhesive and an air-pump bulb 55 for pumping air into the bottle 52. A photograph image of* *Figs.15-1* *and a schematic illustration of* *Fig.15-2* *show a prototype of the powder-spraying device 10'. The air-pump bulb 55 used here is that used for a sphygmomanometer and is "Aneroid Sphygmomanometer premium bladder", which is equipped with a ball valve for back-flow check so that air is pumped in one direction. As shown in* *Fig.15-2**, adopted is a double-wall glass tube 1' that is constructed such that the outer glass tube 12 of the glass tube fixture 1 in* *Figs.2-1 and 2-2* *is modified to have a branch tube 13. The pressure-proof bottle 52 having a cylindrical shape is fitted with a cap 51 formed of silicone rubber; and a double wall part of the double-wall glass tube 1' is attached on the cap 51 as to penetrate center of it.*

In view of uniform spreading of the adhesive, such powder-spraying device 10' equipped with the air-pump bulb is considered to be preferred to the powder-spraying device 10 equipped with the dropper bulb, which is shown in *Figs.2-1 and 2-2**. Moreover, the powder of the adhesive formed of fine particles, such as those having about 30 micrometers of the average particle diameter, might be coagulated in the dropper bulb; when the powder-spraying device 10 equipped with the dropper bulb is used to apply such fine powder of the adhesive.*

### B9. Use in respiratory surgery Obturation of air leakage from lung 2

*Adopted are; the fine powder mixture adhesive obtained in Section B8, and the powder-spraying device 10' in the above. And, the obturation of air leakage was evaluated in a way exactly same as that mentioned in Section B2.*

*For each of the fine powder mixture adhesive and the fibrin glue of "Bolheal", eight trials were made to measure a pressure at which air leakage was observed; and then a range between maximum and minimum and an average value were obtained. Resultantly, for the fine powder mixture adhesive of Section B8, the obtained range is about 40-50cmH₂O and the obtained average value is 46cmH₂O; and, for the fibrin glue of "Bolheal", the obtained range is about 30-40cmH₂O and the obtained average value is 36cmH₂O. As shown in Section 16 of WO*/*06*/*080523; when the two-liquid adhesive was used in place of the powder mixture adhesive, a same manner of experiment revealed that obturation performance of the two part adhesive was in a same level with that of the fibrin glue of "Bolheal". Meanwhile, the fine powder mixture adhesive of Section B8 was used in same manner as the above, for obturation of pinhole of 23G perforated on a surface of a lung; then it was revealed that leakage of air was not observed even when air pressure is raised up to 80cmH₂O.*

*Therefore, the obturation performance achieved by the fine powder mixture adhesive having been obtained at Section B8 was significantly higher than that achieved by the two-liquid adhesive and than that achieved by the fibrin glue. Nevertheless, as mentioned in Section B2, when the powder mixture adhesive having the average particle diameter of 90 micrometers is adopted along with the powder-spraying device 10 equipped with the dropper bulb; observed pressure value of the obturation was slightly smaller than that of the fibrin glue. This is presumably because, due to difference in the particle diameters between two powder mixture adhesives, there was caused a difference in evenness of reaction.*

### B10. On-tissue adherence test

*On-tissue adherence performance or on-tissue bonding performance was evaluated by use of the fine powder mixture adhesive obtained at Section B8. This evaluation was made in accordance with a method written in a report (*Joumal of New Remedies & Clinics, 37(2), 241-245 (1988*)) on an adhesion test results of "Avitene" of Eli Lilly and Company, which is a local haemostatic material, formed of fine fibers of collagen and is commercially available. Modification of the method was made only on a jig. That is, a jig for "a circular patch" was prepared as follows and used for adhesion test. A stainless steel pipe having outer diameter of 12mm and inner diameter of 10mm was cut to a length of 10mm; and then nuts of "M3" were attached by brazing, for forming handgrip portions, on such cut piece of the steel pipe. Subsequently, a metal mesh was attached on a face for adhering, of the cut piece, by soldering. As for the metal mesh, size of the mesh opening was 20mesh*/*inch that is same as in the report; and metal material was modified to brass in view of easiness of metal working.* *Fig.16* *shows photograph images of the jig as seen from above and below and from lateral direction. For evaluation of tissue bonding performance, the jig was placed on a surface of a liver that had been isolated from a rabbit; and then the fine powder mixture adhesive was applied. Applying of the adhesive was made as follows; 0.2g to 0.3g of the fine powder mixture adhesive was taken by a spatula and sprayed through a cylindrical part of the jig; and immediately after such spraying, saline was sprayed. Then, at two minutes after the applying, a horizontal rod, which is connected with a load cell located on upper part of a tensile tester, was inserted through "M3" nuts of the jig and tensile straining was made at a rate of 10mm*/*min.*

*For comparison, the fibrin glue of "Bolheal" and the "Avitene", which is commercially available through Zeria Pharmaceutical Co., Ltd., were used. The "Avitene" is a haemostatic material made from bovine dermal collagen, is formed of fine fibers having diameters of 250-6000nm and lengths of 1-12mm and has white color and a form of strands. Haemostasis mechanism of the "Avitene" is considered as follows; when contacted with bleeding face, blood platelets are seized and coagulation factors of the platelets are activated; and coagulation products are seized in gaps within a fiber network. Applying of the fibrin glue of "Bolheal" was made by use of the fore-mentioned "Bolheal spray set"; and applying of the "Avitene" was made by laying the fine fibers throughout a mesh area of the metal mesh, which forms bottom of the jig.*

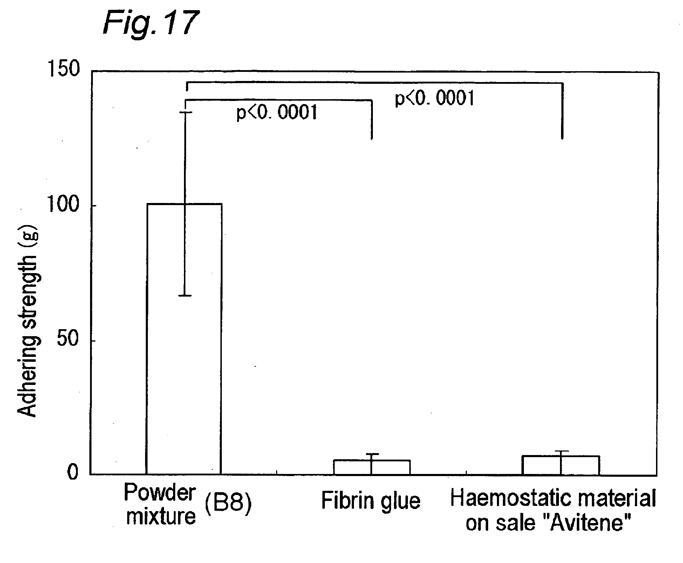

*Consequently, results shown in a graph of* *Fig.17* *were obtained. As indicated in* *Fig.17*, *adhering strength achieved by the powder mixture adhesive of Section B8 was significantly higher than those achieved by the fibrin glue and by the "Avitene"; where p<0.0001. Meanwhile, no significant difference was observed between the fibrin glue and the "Avitene", where p=0.07.*

### <Alpha-glucan aldehyde sheet>

Dextran aldehyde having 0.26 aldehyde groups per anhydroglucose unit (-CHO=0.264±0.003 / sugar residue) was prepared from dextran (Wako Pure Chemical Industries, Ltd; Lot No.EWK3037) having weight-average molecular weight of 75,000, in a method described in Section 1 and Section A1. Then, 15 g of thus obtained dextran aldehyde was mixed with 3.75g of glycerin and 131.25mL of distilled water; and this mixture was kept at 50°C and being agitated as to achieve a solution. The solution was poured as evenly spreaded for cast forming on a polyethylene sheet, which has thickness of 40 micrometers and had been pasted on a glass plate having dimensions of 20X20X0.5cm. Subsequently, such a layer of cast solution was air dried at 25°C for 24 hours, as to form a dextran aldehyde sheet having thickness of about 0.5mm.

As explained by way of an experimental example below, the dextran aldehyde sheet may be used in combination of the two-part adhesive described in the above. In same manner with Section 16, following experiment was made by use of a beagle dog to confirm obturation of the air leakage from lung. In accordance with common procedures, conducted were anesthesia, endotracheal intubation, and then opening of thorax; and a pleural deficient area of 3×3cm was cut out on right lung by use of an electrical scalpel. Physiological saline was sprayed on the deficient area, and pressure of respirator was increased, as to confirm occurrence of air leakage. Subsequently, by use of a mixing device dedicated to mixing of two-part liquids, about 2mL of the adhesive of Reference Example 3 as in Section 16 was dropped on the deficient area. Immediately after the dropping of the adhesive, the dextran sheet that had been cut in a square shape having an area of 5X5cm was pasted on the pleural deficient area. Two minutes later, thorax was filled with physiological saline and a leakage test was made. Resultantly, pressure at which air leakage was observed was 43.5±6.0cmH₂O (sample number n=6, and standard deviation (SD) = 6.0), which is significantly higher than the pressure of 33.3±4.8cmH₂O, which has been obtained without using the dextran sheet.

The dextran aldehyde sheet having thickness in a range of 0.1 to 2mm, preferably 0.3 to 0.8mm may be used in combination with either of two-part adhesives and their usages explained in the above. Thus, the dextran aldehyde sheet may be used for leak stoppage, haemostasis, adhesion, tissue anti-adhesion or the like, in combination with any of the two-liquid adhesives, the liquid-powder adhesives and the two-powder adhesives. In place of the dextran aldehyde sheet, a sheet of other alpha-glucan aldehyde such ad dextrin aldehyde may be used. Density of aldehyde groups per anhydroglucose unit is in a range of 0.1 to 1.0, preferably 0.2 to 0.9, still more preferably 0.3 to 0.8.

### Brief Description of the Several Views of the Drawing

Fig*.1-1* is a photomicrograph showing powder-form first part of the adhesive;
*Fig.1-2* *a graph showing long-time preservability of dextran aldehyde aqueous solution as evaluated by variation of gelling time;*
Fig.2*-1* is a photograph showing a hand-made powder-spraying device prepared by use of a dropper cap;
Fig.*2-2* is a schematic illustration of the *hand-made powder-spraying device*;
Fig. *3* is a photograph showing that bleeding on liver has been arrested;
Fig.*4* is a photograph *showing* that tissue adhesion has been curbed by the powder-liquid two-part adhesive of the embodiments;
Fig.*5* is a photograph in a manner of Fig.*4*, showing that tissue adhesion has been curbed by marketed Seprafilm;
Fig.*6* is a photograph in a manner of Fig.*4*, showing tissue adhesion when no adhesion barrier membrane was used;
Fig.*7* is a photograph showing that air leakage through a pleural deficient area from a lung has been curbed by the *powder mixture two-part* adhesive;
*Fig.8* *is a schematic perspective view of a syringe pump device and a manner of its use, for evaluating pinhole obliteration ;*
*Fig*.*9* *is a graph showing results of evaluation on the pinhole obliteration, along with results by the fibrin glue as a comparison;*
Fig.*10* is a photograph showing cut-opened abdomen of a rat, after a hydrogel layer of cured adhesive has been implanted and left there for a week;

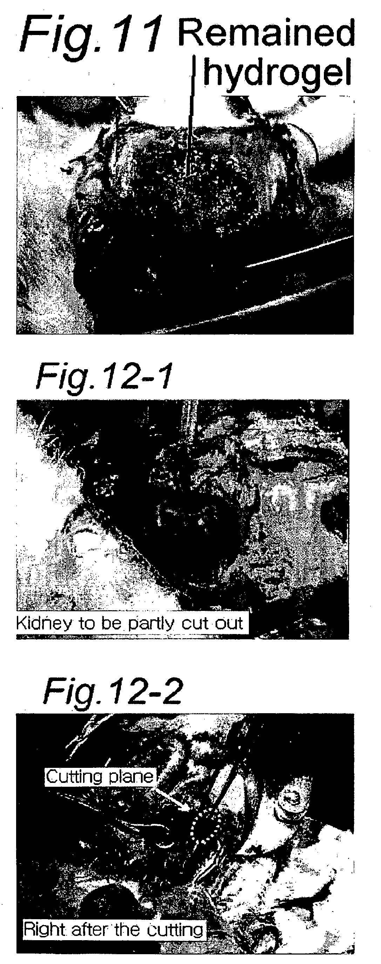

*Fig.11* *is a photograph in a manner of* *Fig.10**, for an occasion the spread of the adhesive was increased and resultantly, remnant of the hydrogel resin layer was observed;*
Fig.*12-1* to *12-3* are a set of photographs indicating a process flow of partially cutting of kidney of a rabbit and arresting of bleeding from cut-out face;
*Fig.13* *is a graph showing evaluation results on adhesion barrier performance of various medical adhesives and an adhesion barrier membrane;*
*Fig.14* *is a microscopic photograph in a manner of* *Fig.1-1**, showing an image of the fine powder mixture adhesive having a smaller average particle diameter;*
*Fig.15-1* *is a photograph in a manner of* *Fig.2-1**, showing a powder-spraying device equipped with an air-pump bulb;*
*Fig.15-2* *is a schematic illustration in a manner of* *Fig.2-2**, showing the powder-spraying device of* *Fig.15-1**;*
*Fig.16* *is a photograph image of a jig used for measuring of adherence strength; and*
*Fig.17* *is a graph showing results of measuring the adherence strength, along with results for a marketed fibrin glue and a marketed haemostatic sheet for comparison.*

## Claims

1. Medical-use two-component adhesive formed of a powder mixture of a first part comprised of powder of aldehyde-groups-introduced alpha-glucan having a weight-average molecular weight in a range of 1000 to 200,000 and a second part comprised of powder of poly-L-lysine that has a molecular weight in a range of 1000 to 20,000,
wherein
the aldehyde-groups-introduced alpha-glucan is produced by oxidation of dextran or dextrin, with periodic acid or periodate, as to have aldehyde groups at a density of 0.1 to 1.0 per anhydroglucose unit,
the molar ratio of aldehyde groups to amino groups in the mixture of the first and second parts is in a range of 0.9 to 3.5,
the powder of the aldehyde-groups-introduced alpha-glucan is porous and has random shapes of particles and an average particle diameter of 10-150 micrometers,
the second part is a powder obtained by freeze drying of aqueous solution of poly-L-lysine added with carboxylic acid and mechanical pulverization of freeze dried product, and average particle diameter of the powder of the second part is in a range of 10-150 micrometers, and
the mixture has a water content of not more than 2.0% and pH in a range of 5.0 to 8.0 when dissolved in water.

2. Medical-use two-component adhesive according to claim 1, wherein the powder of the aldehyde-groups-introduced alpha-glucan is obtained by freeze drying of aqueous solution and mechanical pulverization of freeze dried product.

3. Medical-use two-component adhesive according to claim 1 or 2, wherein the second part is added with acetic acid, citric acid, succinic acid, glutar acid, malic acid, fumaric acid, maleic acid, or other monocarboxylic acid or polycarboxylic acid, or anhydride corresponding at least one of these acids; by 1 to 10wt%.

4. Medical-use two-component adhesive according to anyone of claims 1-3, wherein the poly-L-lysine is epsilon-poly-L-lysine produced by microorganism or by enzyme.

## Patentansprüche

1. Zweikomponentenklebstoff für die medizinische Verwendung, gebildet aus einem Pulvergemisch eines ersten Teils, der Pulver von alpha-Glucan mit eingeführten Aldehydgruppen eines Gewichtsmittel des Molekulargewichts im Bereich von 1000 bis 200.000 enthält, und eines zweiten Teils, der Pulver von Poly-L-lysin mit einem Molekulargewicht im Bereich von 1000 bis 20.000 enthält, wobei
das alpha-Glucan mit eingeführten Aldehydgruppen durch Oxidation von Dextran oder Dextrin mit Periodsäure oder Periodat hergestellt worden ist, so dass Aldehydgruppen in einer Dichte von 0,1 bis 1,0 pro Anhydroglucoseeinheit vorhanden sind,
das Molverhältnis von Aldehydgruppen zu Amingruppen in dem Gemisch des ersten und des zweiten Teils im Bereich von 0,9 bis 3,5 liegt,
das Pulver des alpha-Glucans mit eingeführten Aldehydgruppen porös ist und willkürliche Teilchenformen und einen mittleren Teilchendurchmesser von 10 bis 150 µm aufweist,
der zweite Teil ein Pulver ist, das durch Gefriertrocknen einer wäßrigen Lösung von mit Carbonsäure versetztem Poly-L-lysin und mechanisches Pulverisieren des gefriergetrockneten Produkts erhalten worden ist, und wobei der mittlere Teilchendurchmesser des Pulvers des zweiten Teils im Bereich von 10 bis 150 µm liegt,
und
das Gemisch einen Wassergehalt von nicht mehr als 2,0 % und einem pH-Wert in einem Bereich von 5,0 bis 8,0, wenn es in Wasser gelöst ist, aufweist.

2. Zweikomponentenklebstoff für die medizinische Verwendung nach Anspruch 1, wobei das Pulver des alpha-Glucans mit eingeführten Aldehydgruppen durch Gefriertrocknen einer wäßrigen Lösung und mechanisches Pulverisieren des gefriergetrockneten Produkts erhalten wird.

3. Zweikomponentenklebstoff für die medizinische Verwendung nach Anspruch 1 oder 2, wobei der zweite Teil mit Essigsäure, Zitronensäure, Bernsteinsäure, Glutarsäure, Äpfelsäure, Fumarsäure, Maleinsäure oder einer anderen Monocarbonsäure oder Polycarbonsäure oder mit einem zumindest einer dieser Säuren entsprechenden Anhydrid zu 1 bis 10 Gew.-% versetzt ist.

4. Zweikomponentenklebstoff für die medizinische Verwendung nach einem der Ansprüche 1 bis 3, wobei das Poly-L-lysin durch einen Mikroorganismus oder ein Enzym hergestelltes epsilon-Poly-L-lysin ist.

## Revendications

1. Adhésif à deux composants à usage médical formé d'un mélange de poudres d'une première partie composée d'une poudre d'alpha-glucane dans lequel sont introduits des groupes aldéhyde ayant un poids moléculaire moyen en poids dans une plage de 1 000 à 200 000 et une deuxième partie composée d'une poudre de poly-L-lysine qui a un poids moléculaire dans une plage de 1 000 à 20 000,
dans lequel
l'alpha-glucane dans lequel sont introduits des groupes aldéhyde est produit par oxydation de dextrane ou de dextrine, avec de l'acide périodique ou du périodate, de manière à obtenir des groupes aldéhyde à une densité de 0,1 à 1,0 par unité anhydroglucose,
le rapport molaire des groupes aldéhyde aux groupes amino dans le mélange des première et deuxième parties est dans une plage de 0,9 à 3,5,
la poudre de l'alpha-glucane dans lequel sont introduits des groupes aldéhyde est poreuse et présente des formes aléatoires de particules et un diamètre moyen des particules de 10 à 150 micromètres,
la deuxième partie est une poudre obtenue par lyophilisation d'une solution aqueuse de poly-L-lysine à laquelle est ajouté de l'acide carboxylique et pulvérisation mécanique du produit lyophilisé, et le diamètre moyen des particules de la poudre de la deuxième partie est dans une plage de 10 à 150 micromètres, et
le mélange a une teneur en eau non supérieure à 2,0 % et un pH dans une plage de 5,0 à 8,0 lorsqu'il est dissous dans l'eau.

2. Adhésif à deux composants à usage médical selon la revendication 1, dans lequel la poudre de l'alpha-glucane dans lequel sont introduits des groupes aldéhyde est obtenue par lyophilisation d'une solution aqueuse et pulvérisation mécanique du produit lyophilisé.

3. Adhésif à deux composants à usage médical selon la revendication 1 ou 2, dans lequel on ajoute à la deuxième partie de l'acide acétique, de l'acide citrique, de l'acide succinique, de l'acide glutarique, de l'acide malique, de l'acide fumarique, de l'acide maléique ou un autre acide monocarboxylique ou acide polycarboxylique, ou un anhydride correspondant à au moins l'un de ces acides ; à raison de 1 à 10 % en poids.

4. Adhésif à deux composants à usage médical selon l'une quelconque des revendications 1 à 3, dans lequel la poly-L-lysine est une epsilon-poly-L-lysine produite par un microorganisme ou par une enzyme.
